# EUROPEAN PATENT APPLICATION

(11) **EP 4 585 374 A1**
(43) Date of publication of application: **16.07.2025**
(21) Application number: 23892003.7
(22) Date of filing: 15.11.2023
(51) Int. Cl.: B25J 9/00, B25J 9/16, B25J 13/08, B25J 13/00

(54) **ZERO SETTING METHOD FOR WEARABLE DEVICE, AND WEARABLE DEVICE AND ELECTRONIC DEVICE PERFORMING SAME**

(30) Priority: 16.11.2022 KR 20220153478; 27.12.2022 KR 20220186409
(71) Applicant: Samsung Electronics Co., Ltd., Suwon-si, Gyeonggi-do 16677 (KR)
(72) Inventor: PARK, Jiyoung, Suwon-si, Gyeonggi-do 16677 (KR); SONG, Sukhoon, Suwon-si, Gyeonggi-do 16677 (KR)
(74) Representative: Gulde & Partner
(86) International application number: PCT/KR2023/018350
(87) International publication number: WO 2024/106937

(57) **Abstract**

Disclosed are a zero setting method for a wearable device, and a wearable device and an electronic device performing same. The zero setting method performed by the wearable device comprises the steps of: determining whether zero resetting for a first angle sensor that measures an angle of a leg driving frame included in the wearable device is required; and performing a first zero setting process for the first angle sensor by using a second angle sensor that measures an angle change in a first direction and an angle change in a second direction of the leg driving frame, in response to determining that the zero resetting is required. The step for performing the first zero setting process comprises the steps of: determining a first zero location on the basis of the angle change in the first direction and the angle change in the second direction, which are measured by the second angle sensor; and setting zero for the first angle sensor on the basis of the determined first zero location.

## Description

### TECHNICAL FIELD

The present disclosure relates to a zero setting method of a wearable device and the wearable device and an electronic device for performing the zero setting method.

### BACKGROUND ART

A walking assistance device generally refers to a machine or a device that helps a patient unable to walk on their own because of diseases, accidents, or other causes with their walking exercises for rehabilitation treatment. In our current, rapidly aging society, a growing number of people experience inconvenience when walking or have difficulty with normal walking due to malfunctioning joints, and there is increasing interest in walking assistance devices. A walking assistance device is worn on a user's body to assist the user in walking by providing the necessary muscular strength and to induce the user to walk in a normal walking pattern.

### DISCLOSURE OF THE INVENTION

### TECHNICAL SOLUTIONS

According to an embodiment, a wearable device worn on a user's body includes a driving module configured to generate torque applied to the user's body, a leg driving frame configured to relay the generated torque to the user's leg, a thigh fastener that is connected to the leg driving frame and configured to fix the leg driving frame to the user's leg, a sensor module configured to obtain sensor data including the motion information of the wearable device, and a processor configured to perform zero setting based on the sensor data on a sensor included in the wearable device. The sensor module may include a first angle sensor configured to measure an angle of the leg driving frame and a second angle sensor configured to measure an angle change in a first direction and an angle change in a second direction of the leg driving frame based on a reference position in a sensing range. The process may determine a first zero point position based on the angle change in the first direction and the angle change in the second direction measured by the second angle sensor and may perform a first zero setting process for setting a zero point for the first angle sensor based on the determined first zero point position.

The processor may determine whether zero resetting is required based on a state change of the wearable device and may control to perform the first zero setting process in response to a determination that the zero resetting is required.

The processor may determine the first zero point position based on an angle when the user's leg turns from the first direction to the second direction and an angle when the user's leg turns from the second direction to the first direction, which are measured when the user walks while wearing the wearable device.

The first zero setting process may be automatically performed by the wearable device without notifying the user of performing zero setting.

According to an embodiment, a zero setting method performed by a wearable device includes determining whether zero resetting is required for a first angle sensor configured to measure an angle of a leg driving frame included in the wearable device, and, in response to a determination that the zero resetting is required, by using a second angle sensor configured to measure an angle change in a first direction and an angle change in a second direction of the leg driving frame, performing a first zero setting process for the first angle sensor. The performing of the first zero setting process may include determining a first zero point position based on the angle change in the first direction and the angle change in the second direction measured by the second angle sensor and setting a zero point for the first angle sensor based on the determined first zero point position.

According to an embodiment, non-transitory computer-readable storage medium storing instructions that, when executed by a processor, cause the processor to perform the zero setting method.

### BRIEF DESCRIPTION OF DRAWINGS

FIG. 1 is a diagram illustrating an overview of a wearable device worn on a user's body, according to an embodiment.
FIG. 2 is a diagram illustrating an exercise management system including a wearable device and an electronic device, according to an embodiment.
FIG. 3 is a rear schematic diagram illustrating a wearable device according to an embodiment.
FIG. 4 is a left side view of the wearable device according to an embodiment.
FIGS. 5A and 5B are diagrams each illustrating a configuration of a control system of a wearable device, according to an embodiment.
FIG. 6 is a diagram illustrating an interaction between a wearable device and an electronic device, according to an embodiment.
FIG. 7 is a diagram illustrating a configuration of an electronic device according to an embodiment.
FIG. 8 is a diagram illustrating first zero setting using a second angle sensor according to an embodiment.
FIG. 9 is a flowchart illustrating an operation of a zero setting method of a wearable device, according to an embodiment.
FIG. 10 is a diagram illustrating determining a first zero point position based on sensor data obtained from a second angle sensor, according to an embodiment.
FIG. 11 is a flowchart illustrating an operation of a zero setting method performed between an electronic device and a wearable device, according to an embodiment.
FIG. 12 is a flowchart illustrating a first zero setting process that is automatically performed by a wearable device, according to an embodiment.

### BEST MODE FOR CARRYING OUT THE INVENTION

The following detailed structural or functional description is provided as an example only and various alterations and modifications may be made to embodiments. Accordingly, the embodiments are not construed as limited to the disclosure and should be understood to include all changes, equivalents, and replacements within the idea and the technical scope of the disclosure.

As used herein, the singular forms "a", "an", and "the" include the plural forms as well, unless the context clearly indicates otherwise. It will be further understood that the terms "comprises/including" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. Terms, such as those defined in commonly used dictionaries, are to be interpreted as having a meaning that is consistent with their meaning in the context of the relevant art and are not to be interpreted in an idealized or overly formal sense unless expressly so defined herein.

Hereinafter, embodiments will be described in detail with reference to the accompanying drawings. When describing the examples with reference to the accompanying drawings, like reference numerals refer to like elements and a repeated description related thereto will be omitted.

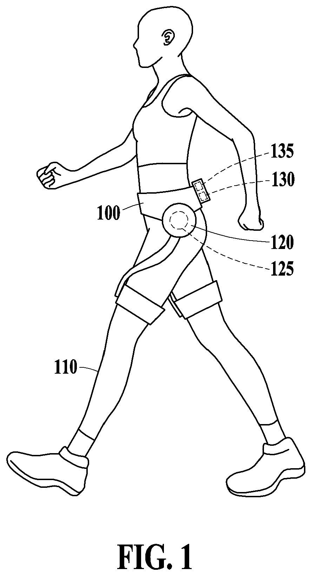

FIG. 1 is a diagram illustrating an overview of a wearable device worn on a user's body, according to an embodiment.

Referring to FIG. 1, in an embodiment, a wearable device 100 may be a device worn on the body of a user 110 to assist the user 110 in walking, exercising, and/or working. In an embodiment, the wearable device 100 may be used to measure a physical ability (e.g., a walking ability, an exercise ability, or an exercise posture) of the user 110. In embodiments, the term "wearable device" may be replaced with "wearable robot," "walking assistance device," or "exercise assistance device". The user 110 may be a human or an animal, but examples are not limited thereto. The wearable device 100 may be worn on the body (e.g., a lower body (legs, ankles, knees, etc.) or a waist) of the user 110 and apply an external force, such as an assistance force and/or a resistance force, to a body motion of the user 110. The assistance force may be a force assisting the body motion of the user 110, which is applied in the same direction as a direction of the body motion of the user 110. The resistance force may be a force impeding the body motion of the user 110, which is applied in an opposite direction to the direction of the body motion of the user 110. The term "resistance force" may also be referred to as an "exercise load".

In an embodiment, the wearable device 100 may operate in a walking assistance mode to assist the user 110 in walking. In the walking assistance mode, the wearable device 100 may assist the walking of the user 110 by applying an assistance force generated through a driving module 120 of the wearable device 100 to the body of the user 110. The wearable device 100 may expand the walking ability of the user 110 by allowing the user 110 to walk independently or walk for a long time by providing a force needed for the walking of the user 110. The wearable device 100 may also improve the walking of a user having an abnormal walking habit or posture.

In an embodiment, the wearable device 100 may operate in an exercise assistance mode to enhance the effect of an exercise on the user 110. In the exercise assistance mode, the wearable device 100 may impede the body motion of the user 110 or resist the body motion of the user 110 by applying a resistance force generated through the driving module 120 of the wearable device 100 to the body of the user 110. When the wearable device 100 is a hip-type wearable device worn on the waist (or pelvis) of the user 110 and the legs (e.g., thighs) of the user 110, the wearable device 100 worn on the legs of the user 110 may enhance the effect of an exercise on the legs of the user 110 by providing an exercise load to the motion of the legs of the user 110. Alternatively, the wearable device 100 may apply an assistance force to the body of the user 110 to assist the exercise of the user 110. For example, when a person with disabilities or an elderly person wears the wearable device 100 to exercise, the wearable device 100 may provide an assistance force to assist body motion in an exercise process. In an embodiment, the wearable device 100 may provide a combination of an assistance force and a resistance force by exercise sessions or time intervals, for example, providing an assistance force in one exercise session and a resistance force in another exercise session.

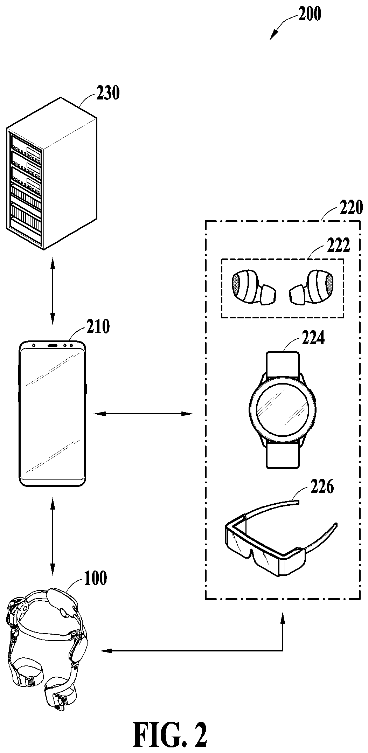

In an embodiment, the wearable device 100 may operate in a physical ability measurement mode (or an exercise ability measurement mode) to measure (including the measurement of an exercise ability) the physical ability of the user 110. The wearable device 100 may measure the motion information of the user by using one or more sensors (e.g., an angle sensor 125 and an inertial measurement unit (IMU) (or an inertia sensor) 135) included in the wearable device 100 while the user 110 walks or exercises. The wearable device 100 and an electronic device (e.g., an electronic device 210 of FIG. 2) interworking with the wearable device 100 may evaluate a physical ability or an exercise ability of the user based on the measured motion information. For example, a gait index or an exercise ability indicator (e.g., muscular strength, endurance, balance, or exercise motion) of the user 110 may be estimated based on the motion information of the user 110 measured by the wearable device 100. The physical ability measurement mode may include an exercise motion evaluation mode to evaluate an exercise motion (or exercise posture) of the user while the user performs an exercise.

In embodiments of the present disclosure, for convenience of description, the wearable device 100 is described as an example of a hip-type wearable device, as illustrated in FIG. 1, but embodiments are not limited thereto. As described above, the wearable device 100 may be worn on another body part (e.g., the upper arms, lower arms, hands, calves, and feet) other than the waist and legs (particularly, the thighs), and the shape and configuration of the wearable device 100 may vary depending on the body part on which the wearable device 100 is worn.

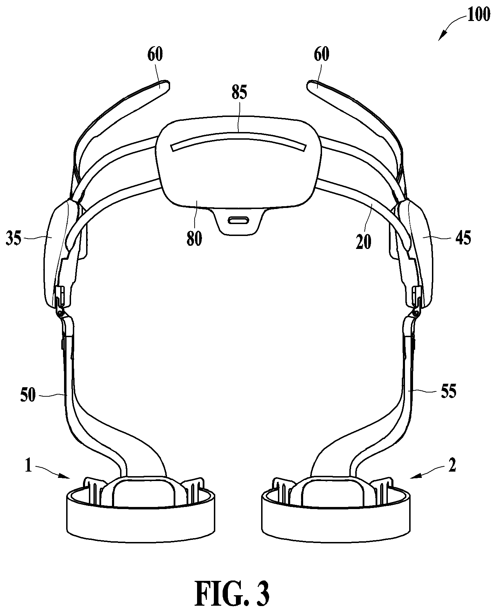

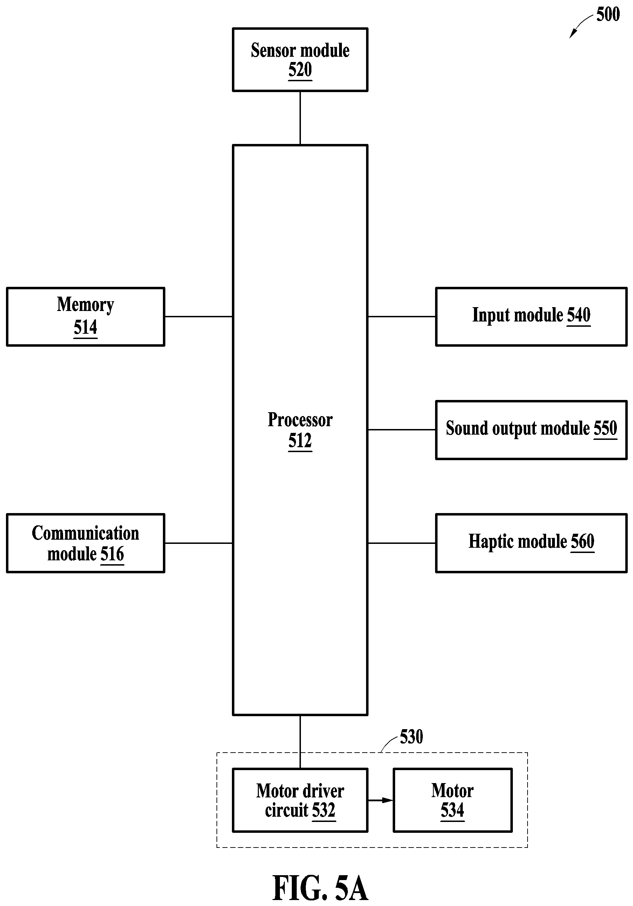

According to an embodiment, the wearable device 100 may include a support frame (e.g., a waist support frame 20 of FIG. 3) for supporting the body of the user 110 when the wearable device 100 is worn on the body of the user 110, the driving module 120 (e.g., driving modules 35 and 45 of FIG. 3) for generating torque applied to the legs of the user 110, a leg driving frame (e.g., first and second leg driving frames 55 and 50 of FIG. 3) for relaying the torque generated by the driving module 120 to the legs of the user 110, a sensor module (e.g., a sensor module 520 of FIG. 5A) including one or more sensors for obtaining sensor data including motion information on the body motion (e.g., leg motion or upper body motion) of the user 110, and a processor 130 for controlling the operation of the wearable device 100.

The sensor module may include the angle sensor 125 and the inertia sensor 135. The angle sensor 125 may measure the rotation angle of the leg driving frame of the wearable device 100 corresponding to a hip joint angle value of the user 110. The rotation angle of the leg driving frame measured by the angle sensor 125 may be estimated as the hip joint angle value (or a leg angle value) of the user 110. The angle sensor 125 may include, for example, an encoder and/or a hall sensor. In an embodiment, the angle sensor 125 may be near a position where a motor included by the driving module 120 is connected to the leg driving frame. The inertia sensor 135 may include an acceleration sensor and/or an angular velocity sensor and may measure changes in acceleration and/or angular velocity according to the motion of the user 110. The inertia sensor 135 may measure a motion value of a base body (e.g., a base body 80 of FIG. 3) or the waist support frame of the wearable device 100, for example. The motion value of the base body or the waist support frame measured by the inertia sensor 135 may be estimated as an upper body motion value of the user 110.

In an embodiment, the processor 130 and the inertia sensor 135 may be inside the base body (e.g., the base body 80 of FIG. 3) of the wearable device 100. The base body may be on the waist (or a lumbar region) of the user 110 when the user 110 wears the wearable device 100. The base body may be formed on or attached to the outside of the waist support frame of the wearable device 100. The base body may be mounted on the lumbar region of the user 110 to provide a cushioning feeling to the lower back of the user 110 and may support the lower back of the user 110 together with the waist support frame.

When the wearable device 100 applies an assistance force or a resistance force to the body of the user 110, the wearable device 100 may apply an assistance force or a resistance in the direction in which a joint of the user 110 moves. In this case, the magnitude of force generated by the wearable device 100 or the effect of an exercise of the user 110 may vary depending on the zero point position of a sensor included in the wearable device 100. In addition, the current posture or exercise posture of the user 110 may be determined differently depending on the zero point position. The magnitude of force applied to the user 110 or the motion assistance scheme may depend on a body motion (e.g., joint motion) of the user 110 measured based on a zero set position. If a zero point is not set or the zero point is set to an incorrect zero point position, abnormal torque may be relayed to the user 110. As such, it is important to accurately set the zero point of the sensor included in the wearable device 100. An initialization process for setting an absolute zero point position is important because the wearable device 100 uses a relative angle value of a joint for the control of a driving module or the determination (e.g., the determination of a walking phase) of a state.

A method of performing zero setting on a sensor of the wearable device 100 includes a method (which is referred to as a "second zero setting process" herein) of performing zero setting based on a sensor value measured from a reference posture after requesting the user 110 to wear the wearable device 100 and take the reference posture (e.g., a standing posture) (or reference motion). This zero setting method may undermine usability because it requires the participation of a user in every zero setting process and incurs the user's time for zero setting. In addition, if the user 110 takes an incorrect reference posture, zero setting through the second zero setting process may result in the zero point being set to an incorrect position.

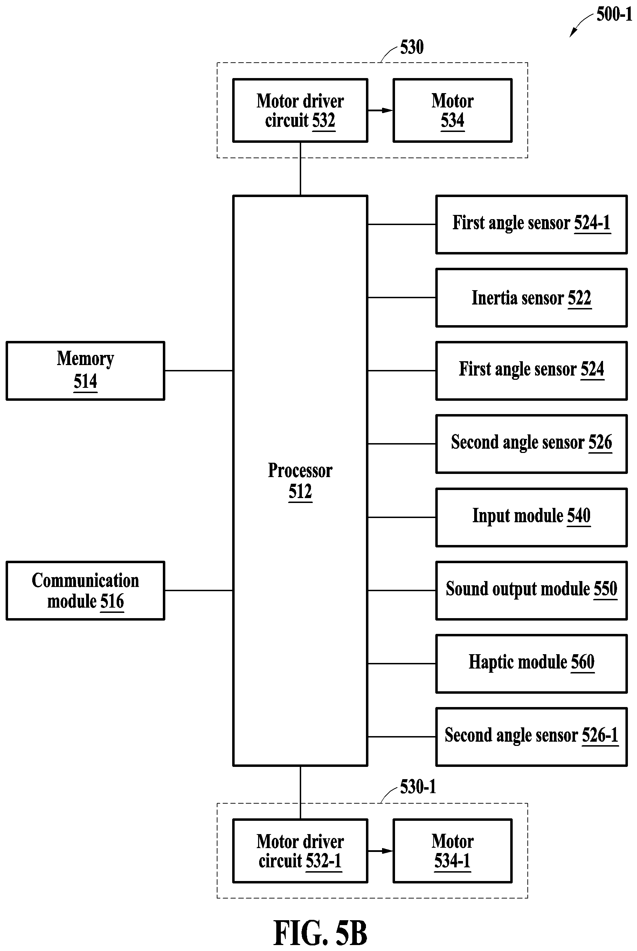

According to various embodiments herein, the zero setting method of the wearable device 100 to improve the user's convenience may be provided. The wearable device 100 may determine whether a zero setting process is required based on the state of the wearable device 100 and may perform the zero setting process only when the zero setting process is determined to be required. In addition, the wearable device 100 may automatically perform the zero setting process (referred to as a "first zero setting process" herein) based on an angle sensor (e.g., a second angle sensor 526 or 526-1 of FIG. 5B) for measuring an angle change of a leg driving frame of the wearable device 100 based on a reference position of the wearable device 100. The wearable device 100 may attempt zero setting through the second zero setting process when zero setting has been determined to be required and the zero setting has not been completed in the first zero setting process. As such, the wearable device 100 may improve the convenience of a user by automatically determining whether zero setting is required to reduce the number of performing zero setting being performed and preferentially performing the first zero setting process automatically performed by the wearable device 100.

FIG. 2 is a diagram illustrating an exercise management system including a wearable device and an electronic device, according to an embodiment.

Referring to FIG. 2, an exercise management system 200 may include the wearable device 100, the electronic device 210, another wearable device 220, and a server 230. In an embodiment, at least one (e.g., the other wearable device 220 or the server 230) of these devices may be omitted from the exercise management system 200, or one or more other devices (e.g., an exclusive controller device of the wearable device 100) may be added to the exercise management system 200.

In an embodiment, the wearable device 100 worn on the body of a user may assist the motion of the user in a walking assistance mode. For example, the wearable device 100 worn on the user's legs may assist the user in walking by generating an assistance force to assist the motion of the user's legs.

In an embodiment, to enhance an exercise effect on the user in an exercise assistance mode, the wearable device 100 may generate a resistance force to impede the body motion of the user or an assistance force to assist the body motion of the user and may apply such a force to the body of the user. In the exercise assistance mode, through the electronic device 210, the user may select an exercise program (e.g., squat, split lunge, dumbbell squat, lunge and knee up, stretching, etc.) that the user desires to do for exercise by using the wearable device 100 and/or exercise intensity to be applied to the wearable device 100. The wearable device 100 may control a driving module of the wearable device 100 according to the exercise program selected by the user and may obtain sensor data including motion information of the user through a sensor module. The wearable device 100 may adjust the intensity of a resistance force or an assistance force applied to the user according to the exercise intensity selected by the user. For example, the wearable device 100 may control the driving module to generate a resistive force corresponding to the exercise intensity selected by the user.

In an embodiment, the wearable device 100 may be used to measure the physical ability of the user by interoperating with the electronic device 210. This measurement of physical ability may include the measurement of an exercise ability. The wearable device 100 may operate in a physical ability measurement mode, which is a mode for measuring the physical ability of the user, by control of the electronic device 210, and may transmit sensor data obtained by the motion of the user in the physical ability measurement mode to the electronic device 210. The wearable device 100 may transmit the user's motion data in real time to the electronic device 210. The electronic device 210 may evaluate the physical ability of the user by analyzing the sensor data received from the wearable device 100.

The electronic device 210 may communicate with the wearable device 100 and may remotely control the wearable device 100 or provide the user with state information on a state (e.g., a booting state, a charging state, a sensing state, or an error state) of the wearable device 100. The electronic device 210 may receive sensor data obtained by a sensor module of the wearable device 100 from the wearable device 100 and may estimate the physical ability or exercise results of the user based on the received sensor data. In an embodiment, the electronic device 210 may provide the physical ability or exercise results of the user to the user through a graphical user interface (GUI).

In an embodiment, the user may execute a program (e.g., an application) in the electronic device 210 to control the wearable device 100 and may adjust torque intensity output from the driving module (e.g., the driving modules 35 and 45 of FIG. 3), audio intensity output from a sound output module (e.g., a sound output module 550 of FIGS. 5A and 5B), and brightness of a lighting unit (e.g., a lighting unit 85 of FIG. 3), based on a set value or an operation of the wearable device 100 through the program. The program executed by the electronic device 210 may provide the GUI for an interaction with the user. The electronic device 210 may be a device in various forms. For example, the electronic device 210 may include a portable communication device (e.g., a smartphone), a computer device, an access point, a portable multimedia device, or a home appliance (e.g., a television, an audio device, or a projector device), but examples are not limited to the foregoing devices.

In an embodiment, the electronic device 210 may be connected to the server 230 by using short-range wireless communication or cellular communication. The server 230 may receive user profile information of the user of the wearable device 100 from the electronic device 210 and store and manage the received user profile information. The user profile information may include, for example, information about at least one of the name, age, gender, height, weight, exercise goal, physical fitness level, medical history, or body mass index (BMI). The server 230 may receive exercise history information on an exercise performed by the user from the electronic device 210 and may store and manage the received exercise history information. The server 230 may provide the electronic device 210 with various exercise programs or physical ability measurement programs to be provided to the user. For example, the server 230 may be a cloud server. In an embodiment, the motion data measured by the wearable device 100 may be transmitted to the server 230 via the electronic device 210, and the server 230 may analyze the physical ability or exercise results of the user based on the user's motion data. Result data derived from the analysis of the server 230 may be transmitted to the electronic device 210.

In an embodiment, the wearable device 100 and/or the electronic device 210 may be connected to the other wearable devices 220. The other wearable devices 220 may include, for example, wireless earphones 222, a smartwatch 224, or smart glasses 226, but examples are not limited to the foregoing devices. The wireless earphones 222 may be linked with the electronic device 210 to provide auditory feedback to the user. The wireless earphones 222 may output, for example, a guide voice for guiding the proper wearing of the wearable device 100 and for guiding actions for sensor initialization and/or a guide voice for real-time exercise coaching to the user. In an embodiment, the wireless earphones 222 may also function as a microphone for voice recognition. The user may control the electronic device 210 and the wearable device 100 through voice recognition.

In an embodiment, the smartwatch 224 may measure a biosignal including heart rate information of the user, and may transmit the measured biosignal to the electronic device 210 and/or the wearable device 100. The electronic device 210 may estimate the heart rate information (e.g., a current heart rate, a maximum heart rate, or an average heart rate) of the user, based on the biosignal received from the smartwatch 224, and may provide the estimated heart rate information to the user. In an embodiment, the smartwatch 224 may output a guide screen for guiding exercise coaching and/or an exercise performance method to the user while the user is exercising.

In an embodiment, the exercise result information, physical ability information, and/or exercise motion evaluation information of the user that is determined by the electronic device 210 may be transmitted to the other wearable devices 220 and provided to the user through the other wearable devices 220. The state information of the wearable device 100 may be transmitted to the other wearable devices 220 and may be provided to the user through the other wearable devices 220. For example, the exercise result information may be displayed on the screen of the smartwatch 224, or a guide voice for guiding the exercise result information may be output through the wireless earphones 222. In an embodiment, the wearable device 100, the electronic device 210, and the other wearable devices 220 may be connected to one another through wireless communication (e.g., Bluetooth^{™} or Wi-Fi communication).

In an embodiment, the wearable device 100 may provide (or output) feedback (e.g., visual, auditory, or tactile feedback) corresponding to the state of the wearable device 100 according to a control signal received from the electronic device 210. For example, the wearable device 100 may provide visual feedback through the lighting unit (the lighting unit 85 of FIG. 3) and auditory feedback through the sound output module (e.g., the sound output module 550 of FIGS. 5A and 5B). The wearable device 100 may provide haptic feedback in the form of vibration to the body of the user through a haptic module (e.g., a haptic module 560 of FIGS. 5A and 5B). The electronic device 210 may also provide (or output) feedback (e.g., visual feedback, auditory feedback, or haptic feedback) corresponding to the state of the wearable device 100. In an embodiment, the electronic device 210 may perform a real-time voice-based exercise coaching function while the user is exercising and may provide relevant information to the user through various feedback means.

In an embodiment, the electronic device 210 may present a personalized exercise goal to the user in the exercise assistance mode or the physical ability measurement mode. The personalized exercise goal may include a target amount of exercise for each exercise type (e.g., strength exercise, balance exercise, and aerobic exercise) desired by the user, which is determined by the electronic device 210 and/or the server 230. When the server 230 determines a target amount of exercise, the server 230 may transmit information about the determined target amount of exercise to the electronic device 210. The electronic device 210 may personalize and present the target amount of exercise for each exercise type, such as strength exercise, aerobic exercise, and balance exercise, according to a desired exercise program (e.g., squat, split lunge, or lunge and knee up) and/or the physical characteristics (e.g., the age, height, weight, and BMI) of the user. The electronic device 210 may display a GUI screen indicating an exercise amount target value of each exercise type on a display or may guide the user to the exercise amount target value through a guide voice.

In an embodiment, the electronic device 210 and/or the server 230 may include a database in which information about a plurality of exercise programs to be provided to the user through the wearable device 100 is stored. To achieve an exercise goal of the user, the electronic device 210 and/or the server 230 may recommend an exercise program suitable for the user. The exercise goal may include, for example, at least one of muscle strength improvement, physical strength improvement, cardiovascular endurance improvement, core stability improvement, flexibility improvement, or symmetry improvement. The electronic device 210 and/or the server 230 may store and manage the exercise program performed by the user, the results of performing the exercise program, and the like. Exercise programs recommended to the user may be guided to the user through a guide voice or may be displayed through the GUI screen.

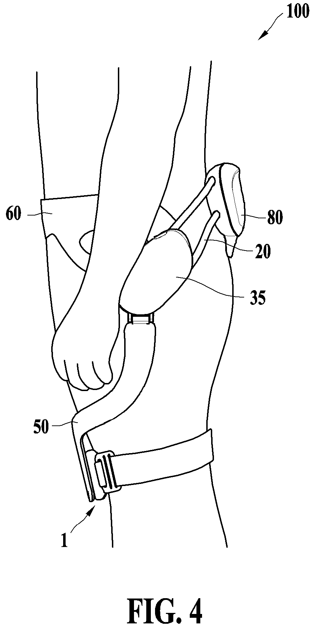

FIG. 3 is a rear schematic diagram illustrating a wearable device according to an embodiment. FIG. 4 is a left side view of the wearable device according to an embodiment.

Referring to FIGS. 3 and 4, a wearable device 100 may include the base body 80, the waist support frame 20, the driving modules 35 and 45, the first and second leg driving frames 55 and 50, first and second thigh fasteners 2 and 1, and a waist fastener 60. The base body 80 may include the lighting unit 85. In an embodiment, at least one (e.g., the lighting unit 85) of the components may be omitted from the wearable device 100, or one or more other components may be added to the wearable device 100.

The base body 80 may be on the waist of a user when the user wears the wearable device 100. The base body 80 worn on the waist of the user may cushion and support the waist of the user. The base body 80 may be above the hip of the user when the user wears the wearable device 100 such that the wearable device 100 may not deviate downward due to gravity. The base body 80 may distribute some of the weight of the wearable device 100 to the waist of the user while wearing the wearable device 100. The base body 80 may be connected to the waist support frame 20. Waist support frame connection elements (not shown) which may be connected to the waist support frame 20 may be at both edges of the base body 80.

In an embodiment, the lighting unit 85 may be on the outer surface of the base body 80. The lighting unit 85 may include a light source (e.g., light emitting diode (LED)). The lighting unit 85 may emit light by control of a processor (not shown) (e.g., a processor 512 of FIGS. 5A and 5B). According to embodiments, the processor may control the lighting unit 85 to provide (or output) visual feedback corresponding to the state of the wearable device 100.

The waist support frame 20 may support the body (e.g., the waist) of the user when the wearable device 100 is worn on the body of the user. The waist support frame 20 may extend from both edges of the base body 80. The waist of the user may be accommodated inside the waist support frame 20. The waist support frame 20 may include one or more rigid body beams. Each beam may be a curved shape with a preset curvature such that the beam may enclose the user's waist. The waist fastener 60 may be connected to an edge of the waist support frame 20. The driving modules 35 and 45 may be connected to the waist support frame 20.

In an embodiment, the processor, a memory, an inertia sensor (e.g., the inertia sensor 135 of FIG. 1 or an inertia sensor 522 of FIG. 5B), a communication module (e.g., a communication module 516 of FIGS. 5A and 5B), a sound output module (e.g., the sound output module 550 of FIGS. 5A and 5B) and a battery (not shown) may be inside the base body 80. The base body 80 may protect the components inside the base body 80. The processor may generate a control signal to control the operation of the wearable device 100. The processor may control an actuator of the driving modules 35 and 45. The processor and the memory may be included by a control circuit. The control circuit may further include a power supply circuit to provide battery power to each component of the wearable device 100.

In an embodiment, the wearable device 100 may include a sensor module (not shown) (e.g., the sensor module 520 of FIG. 5A) for obtaining sensor data from one or more sensors. The sensor module may obtain sensor data including the motion information of components of the wearable device 100 and/or the motion information of the user. The sensor module may include, for example, the inertia sensor (e.g., the inertia sensor 135 of FIG. 1 or the inertia sensor 522 of FIG. 5B) for measuring an upper body motion value of the user or a motion value of the waist support frame 20 and an angle sensor (e.g., the angle sensor 125 of FIG. 1, a first angle sensor 524, a first angle sensor 524-1, the second angle sensor 526, and the second angle sensor 526-1 of FIG. 5B) for measuring a hip joint angle value of the user or a motion value of the first and second leg driving frames 55 and 50, but examples are not limited thereto. For example, the sensor module may further include at least one of a position sensor, a temperature sensor, a biosignal sensor, and a proximity sensor.

The waist fastener 60 may be connected to the waist support frame 20 and may fix the waist support frame 20 to the waist of the user. The waist fastener 60 may include, for example, a pair of belts.

The driving modules 35 and 45 may generate an external force (or torque) to be applied to the body of the user based on the control signal generated by the processor. For example, the driving modules 35 and 45 may generate an assistance force or a resistance force to be applied to the user's legs. In an embodiment, the driving modules 35 and 45 may include a first driving module 45 in a position corresponding to a position of a right hip joint of the user and a second driving module 35 in a position corresponding to a position of a left hip joint of the user. The first driving module 45 may include a first actuator and a first joint member, and the second driving module 35 may include a second actuator and a second joint member. The first actuator may provide power to be relayed to the first joint member, and the second actuator may provide power to be relayed to the second joint member. The first actuator and the second actuator may respectively include motors (motors 534 and 534-1 of FIG. 5B) for generating power (or torque) by receiving power from the battery. When the motor receives power and is driven, the motor may generate a force (an assistance force) for assisting a body motion of the user or a force (a resistance force) for hindering a body motion of the user. In an embodiment, the processor may adjust the magnitude or direction of a force generated by the motor by adjusting a voltage or a current supplied to the motor.

In an embodiment, the first joint member and the second joint member may receive power respectively from the first actuator and the second actuator and may apply an external force to the body of the user based on the received power. The first joint member and the second joint member may respectively be at positions corresponding to the joints of the user. A side of the first joint member may be connected to the first actuator, and the other side of the first joint member may be connected to the first leg driving frame 55. The first joint member may rotate by the power relayed from the first actuator. An encoder or a hall sensor that may operate as an angle sensor to measure a rotation angle (corresponding to a joint angle of the user) of the first joint member or the first leg driving frame 55 may be on a side of the first joint member. A side of the second joint member may be connected to the second actuator, and the other side of the second joint member may be connected to the second leg driving frame 50. The second joint member may rotate by the power relayed from the second actuator. An encoder or a hall sensor that may operate as an angle sensor to measure a rotation angle of the second joint member or the second leg driving frame 50 may be on a side of the second joint member.

In an embodiment, the first actuator may be in a lateral direction of the first joint member, and the second actuator may be in a lateral direction of the second j oint member. A rotation axis of the first actuator may be spaced apart from a rotation axis of the first joint member, and a rotation axis of the second actuator may also be spaced apart from a rotation axis of the second joint member. However, examples are not limited to the foregoing examples, an actuator and a joint member may share a rotation axis. In an embodiment, actuators may respectively be spaced apart from joint members. In this case, the driving modules 35 and 45 may further include a power transmission module (not shown) for relaying power from the actuators to the joint members. The power transmission module may be a rotary body, such as a gear, or a longitudinal member, such as a wire, a cable, a string, a spring, a belt, or a chain. However, the scope of embodiments is not limited by a power relay structure and a positional relationship between the actuators and joint members described above.

In an embodiment, the first and second leg driving frames 55 and 50 may relay torque generated by the driving modules 35 and 45 to the body (e.g., the thighs) of the user when the wearable device 100 is worn on the user's legs. The relayed torque may function as an external force applied to a motion of the user's legs. An edge of the first and second leg driving frames 55 and 50 may be connected to a joint member and rotate, and the other edge of the leg driving frames 50 and 55 may be connected to the first and second thigh fasteners 2 and 1 and relay the torque generated by the driving modules 35 and 45 to the user's thighs while supporting the user's thighs. For example, the first and second leg driving frames 55 and 50 may push or pull the user's thighs. The first and second leg driving frames 55 and 50 may extend in a longitudinal direction of the user's thighs. The leg driving frames 50 and 55 may be folded to wrap around at least a portion of the thigh circumference of the user. The leg driving frames 50 and 55 may include a first leg driving frame 55 for relaying torque to a right leg of the user and a second leg driving frame 50 for relaying torque to a left leg of the user.

The first and second thigh fasteners 2 and 1 are respectively connected to the first and second leg driving frames 55 and 50 and may fix the wearable device 100 to the user's thighs. For example, the first and second thigh fasteners 2 and 1 may include the first thigh fastener 2 to fix the wearable device 100 to a right thigh of the user and the second thigh fastener 1 to fix the wearable device 100 to a left thigh of the user.

In an embodiment, the first thigh fastener 2 may include a first cover, a first fastening frame, and a first strap, and the second thigh fastener 1 may include a second cover, a second fastening frame, and a second strap. The first and second covers may apply torque generated by the driving modules 35 and 45 to the user's thighs. The first and second covers may be on a side of the user's thighs to push or pull the user's thighs. The first and second covers may be on the front surfaces of the user's thighs. The first and second covers may be in a circumferential direction of the user's thighs. The first and second covers may extend to both sides around the other edge of the first and second leg driving frames 55 and 50 and may include curved surfaces corresponding to the user's thighs. An edge of the first and second covers may be connected to a fastening frame and the other edge of the first and second covers may be connected to a strap.

For example, the first and second fastening frames may enclose at least some of the circumference of the user's thighs to prevent the user's thighs from being separated from the wearable device 100. The first fastening frame may have a fastening structure that connects the first cover to the first strap, and the second fastening frame may have a fastening structure that connects the second cover to the second strap.

The first strap may enclose the rest of the circumference of the right thigh of the user, which the first cover and the first fastening frame do not enclose, and the second strap may enclose the rest of the circumference of the left thigh of the user, which the second cover and the second fastening frame do not enclose. The first and second straps may include, for example, elastic material (e.g., a band).

FIGS. 5A and 5B are diagrams each illustrating a configuration of a control system of a wearable device, according to an embodiment.

Referring to FIG. 5A, the wearable device 100 may be controlled by a control system 500. The control system 500 may include the processor 512 (e.g., the processor 130), a memory 514, the communication module 516, the sensor module 520, an input module 530, a sound output module 540, the sound output module 550, and the haptic module 560. In an embodiment, at least one (e.g., the sound output module 550 or the haptic module 560) of those components may be omitted from the control system 500, or one or more other components may be added to the control system 500.

The driving module 530 may include a motor 534 for generating torque (e.g., power) and a motor driver circuit 532 for controlling the motor 534. Although FIG. 5A illustrates the driving module 530 including one motor driver circuit 532 and one motor 534, the example of FIG. 5A is just an example. Referring to FIG. 5B, a control system 500-1 may include two or more (e.g., three or more) motor driver circuits 532 and 532-1 and a plurality of (e.g., two or more) motors 534 and 534-1. The driving module 530 including the motor driver circuit 532 and the motor 534 may correspond to the first driving module 45 of FIG. 3, and a driving module 530-1 including the motor driver circuit 532-1 and the motor 534-1 may correspond to the second driving module 35 of FIG. 3. The following descriptions of the motor driver circuit 532 and the motor 534 may also be respectively applied to the motor driver circuit 532-1 and the motor 534-1 illustrated in FIG. 5B.

Referring to FIG. 5A again, the sensor module 520 may include a sensor circuit including at least one sensor. The sensor module 520 may include sensor data including motion information of components of the wearable device 100 (e.g., the waist support frame 20, the base body 80, or the leg driving frames 50 and 55). In an embodiment, the motion information of the components of the wearable device 100 may correspond to body motion information of a user. The sensor module 520 may transmit the obtained sensor data to the processor 512 or may store it in a separate storage module (not shown) including the memory 514. The sensor module 520 may include the inertial sensor 522 and an angle sensor (e.g., the first angle sensor 524, the first angle sensor 524-1, the second angle sensor 526, or the second angle sensor 526-1).

The inertia sensor 522 (e.g., the inertia sensor 135) may measure an upper body motion value of the user wearing the wearable device 100. For example, the inertia sensor 522 may sense the acceleration and angular velocity of an X-axis, a Y-axis, and a Z-axis according to a motion of the user. The inertia sensor 522 may be used to measure, for example, at least one of a forward and backward tilt, a left and right tilt, or a rotation of the body of the user. In an embodiment, the inertia sensor 522 may obtain a motion value (e.g., an acceleration value and an angular velocity value) of a waist support frame (e.g., the waist support frame 20 of FIG. 3) or a base body (e.g., the base body 80 of FIG. 3) of the wearable device. The motion values of the waist support frame or the base body may correspond to the upper body motion values of the user.

The first angle sensor 524 and the first angle sensor 524-1 may measure a hip joint angle value according to the leg motion of the user. Sensor data that may be sensed by the first angle sensor 524 and the first angle sensor 524-1 may include, for example, a hip joint angle value of a right leg, a hip joint angle value of a left leg, and information on a motion direction of the legs. For example, the first angle sensor 524 of FIG. 5B may obtain the hip joint angle value of the right leg of the user, and the first angle sensor 524-1 may obtain the hip joint angle value of the left leg of the user. The first angle sensor 524 and the first angle sensor 524-1 may each include, for example, an encoder and/or a hall sensor. In addition, the first angle sensor 524 and the first angle sensor 524-1 may obtain a motion value of a leg driving frame of the wearable device 100. For example, the first angle sensor 524 may obtain a motion value of the first leg driving frame 55 of FIG. 3 and the first angle sensor 524-1 may obtain a motion value of the second leg driving frame 50. Motion values of leg driving frames may correspond to hip joint angle values.

The second angle sensor 526 and the second angle sensor 526-1 may measure an angle change in a first direction and an angle change in a second direction of the leg driving frames 50 and 55 based on a reference position within a detection range. The second angle sensor 526 and the second angle sensor 526-1 may be, for example, a hall sensor or an absolute encoder. The second angle sensor 526 may measure the angle change in the first direction (e.g., a forward direction or a flexion direction of a leg) and the angle change in the second direction (e.g., a backward direction or an extension direction of the leg) based on the reference position of the first leg driving frame 55. The second angle sensor 526-1 may measure the angle change in the first direction (e.g., a forward direction or a flexion direction of a leg) and the angle change in the second direction (e.g., a backward direction or an extension direction of the leg) based on the reference position of the second leg driving frame 50. In this case, the reference position may correspond to, for example, an initial position of the leg driving frames 50 and 55 or the position of the leg driving frames 50 and 55 when no force is applied to the leg driving frames 50 and 55.

In an embodiment, the sensor module 520 may further include at least one of a position sensor configured to obtain a position value of the wearable device 100, a proximity sensor configured to sense the proximity of an object, a biosignal sensor configured to detect a biosignal of the user, or a temperature sensor configured to measure an ambient temperature. The types of sensors that may include the sensor module 520 are not limited to the foregoing examples.

The input module 540 may receive a command or data to be used by another component (e.g., the processor 512) of the wearable device 100 from the outside (e.g., the user) of the wearable device 100. The input module 540 may include an input component circuit. The input module 540 may include, for example, a key (e.g., a button) or a touch screen.

The sound output module 550 may output a sound signal to the outside of the wearable device 100. The sound output module 550 may provide auditory feedback to the user. For example, the sound output module 550 may include a speaker configured to play back a guiding sound signal (e.g., an operation start sound, an operation error alarm, or an exercise start alarm), music content, or a guiding voice for auditorily informing predetermined information (e.g., exercise result information or exercise posture evaluation information).

The haptic module 560 may provide haptic feedback to the user by control of the processor 512. The haptic module 560 may convert an electric signal into a mechanical stimulus (e.g., a vibration or a movement) or an electrical stimulus, which may be recognized by a user via their tactile sensation or kinesthetic sensation. The haptic module 560 may include a motor, a piezoelectric element, or an electrical stimulation device. In an embodiment, the haptic module 560 may be positioned in at least one of the base body (e.g., the base body 80) or a thigh fastener (e.g., the first thigh fastener 2 or the second thigh fastener 1).

In an embodiment, the control systems 500 and 500-1 may include a battery (not shown) for supplying power to each component of the wearable device 100 and a power management circuit (not shown) for converting the power of the battery into an operating voltage of each component of the wearable device 100 and supplying the converted power to each component.

The driving module 530 may generate an external force to be applied to the user's leg by control of the processor 512. The driving module 530 may generate torque to be applied to the user's leg based on a control signal generated by the processor 512. The processor 512 may transmit a control signal to the motor driver circuit 532 to control the operation of the motor 534. The motor driver circuit 532 may control the operation of the motor 534 by generating a current signal (or voltage signal) corresponding to the control signal received from the processor 512 and supplying the generated current signal to the motor 534. The current signal may not be supplied to the motor 534 depending on an operation mode of the wearable device 100. When the motor 534 is supplied with the current signal and driven, the motor 534 may generate torque for an assistance force for assisting a leg motion of the user or a resistance force for hindering a leg motion of the user.

The processor 512 may execute software to control at least another component (e.g., a hardware or software component) of the wearable device connected to the processor 512 and may perform various types of data processing or operations. For example, the processor 512 may generate a control signal to control each component (e.g., the communication module 516, the driving module 530, the sound output module 550, or the haptic module 560) of the wearable device 100. The software executed by the processor 512 may include an application for providing a GUI. In an embodiment, as at least part of data processing or operations, the processor 512 may store instructions or data received from another component (e.g., the communication module 516) in the memory 514, may process the instructions or the data stored in the memory 514, and may store result data after the processing in the memory 514. According to an embodiment, the processor 512 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of or in conjunction with the main processor. The auxiliary processor may be implemented separately from the main processor or as a part of the main processor.

The memory 514 may store various pieces of data used by at least one component (e.g., the processor 512) of the wearable device 100. The various pieces of data may include, for example, software, sensor data, input data, or output data for instructions related thereto. The memory 514 may include a volatile memory or a non-volatile memory.

The communication module 516 may support establishing a direct (e.g., wired) communication channel or a wireless communication channel between the processor 512 and another component of the wearable device 100 or an external electronic device (e.g., the electronic device 210 or the other wearable device 220) and performing communication via the established communication channel. The communication module 516 may include a communication circuit configured to perform a communication function. For example, the communication module 516 may receive a control signal from an electronic device (e.g., the electronic device 210) and may transmit the sensor data obtained by the sensor module 520 to the electronic device. According to an embodiment, the communication module 516 may include one or more CPs (not shown) that are operable independently of the processor 512 and that support direct (e.g., wired) communication or wireless communication. In an embodiment, the communication module 516 may include a wireless communication module (e.g., a cellular communication module, a short-range wireless communication module, or a global navigation satellite system (GNSS) communication module) and/or a wired communication module. A corresponding one of these communication modules may communicate with another component of the wearable device 100 and/or an electronic device via a short-range communication network, such as Bluetooth^{™}, wireless-fidelity (Wi-Fi), or infrared data association (IrDA)), or a long-range communication network, such as a legacy cellular network, a 5G network, a next-generation communication network, the Internet, or a computer network (e.g., a local area network (LAN) or a wide area network (WAN)).

According to an embodiment, the wearable device 100 worn on the user's body may include the driving modules 530 and 530-1 for generating torque applied to the user's body. The wearable device 100 may further include the leg driving frames 50 and 55 for relaying the torque generated by the driving modules 530 and 530-1 to the user's leg. The wearable device 100 may further include the thigh fasteners 1 and 2 that are connected to the leg driving frames 50 and 55 and configured to fix the leg driving frames 50 and 55 to the user's leg. The wearable device 100 may further include the sensor module 520 configured to obtain sensor data including the motion information of the wearable device 100. The wearable device 100 may include the processor 512 configured to perform zero setting on a sensor (e.g., the first angle sensor 524 or the first angle sensor 524-1) included in the wearable device 100 based on the sensor data.

In an embodiment, the sensor module 520 may include the first angle sensors 524 and 524-1 configured to measure an angle of the leg drive frames 50 and 55 and the second angle sensors 526 and 526-1 configured to measure an angle change in the first direction and an angle change in the second direction of the leg drive frames 50 and 55 based on the reference position within the detection range. The processor 512 may determine a first zero point position based on the angle change in the first direction and the angle change in the second direction measured by the second angle sensors 526 and 526-1 and may perform a first zero setting process for setting a zero point for the first angle sensors 524 and 524-1 based on the determined first zero point position. In an embodiment, the processor 512 may determine the first zero point position based on an angle when the user's leg turns from the first direction to the second direction and an angle when the user's leg turns from the second direction to the first direction, which are measured when the user walks while wearing the wearable device 100.

For example, the processor 512 may determine the first zero point position corresponding to the first leg driving frame 55 (or the user's right leg) based on an angle change in a forward direction (or a flexion direction) and an angle change in a backward direction (or an extension direction) of the first leg driving frame 55 measured by the second angle sensor 526 when the user wearing the wearable device 100 walks. The processor 512 may initialize the sensor data of the first angle sensor 524 that measures an angle of the first leg driving frame 55 based on the determined first zero point position. In addition, the processor 512 may determine the first zero point position corresponding to the second leg driving frame 50 (or the user's left leg) based on an angle change in a forward direction (or a flexion direction) and an angle change in a backward direction (or an extension direction) of the second leg driving frame 50 measured by the second angle sensor 526-1 when the user wearing the wearable device 100 walks. The processor 512 may initialize the sensor data of the first angle sensor 524-1 that measures an angle of the second leg driving frame 50 based on the determined first zero point position.

In an embodiment, the processor 512 may determine whether zero resetting is required based on a state change of the wearable device 100. The state change of the wearable device 100 may include, for example, a change in at least one of a connection state between the wearable device 100 and a peripheral device (e.g., the electronic device 210, the other wearable device 220, or an access point device), an operation state (e.g., a standby state or an activation state) of the wearable device 100, and a state (e.g., a motor state) of the driving modules 530 and 530-1. In addition, the processor 512 may determine whether zero resetting is required based on whether an error occurs in the wearable device 100. For example, at least one case where a connection with the electronic device 210 is detected, the power of the wearable device 100 is on, the wearable device 100 is changed from a standby state to an activation state, a motor stops for a certain period and starts operating again, an error occurs in operating programs or operations of the wearable device 100, and a change of the user wearing the wearable device 100 is detected, the processor 512 may determine that zero resetting is required. In response to a determination that the zero resetting is required, the processor 512 may control to perform the first zero setting process described above.

In an embodiment, the first zero setting process described above may be automatically performed by the wearable device 100 without notifying the user of performing zero setting. If zero setting is completed through the first zero setting process, the wearable device 100 may initialize the sensor data of the first angle sensor 524 and the first angle sensor 524-1 based on a zero setting result and may start measuring the motion (or the motion of the user's leg) of the leg driving frames 50 and 55.

In an embodiment, the wearable device 100 may further include the communication module 516 that wirelessly communicates with the electronic device 210. For example, the communication module 516 may periodically transmit zero setting state data indicating a zero setting state of the wearable device 100 to the electronic device 210. The electronic device 210 may transmit command data to perform second zero setting to the wearable device 100 if the first zero point position, according to first zero setting, of the wearable device 100 is identified as undetermined, based on the zero setting state data.

When the communication module 516 receives the command data to perform the second zero setting from the electronic device 210, in response to receiving the command data to perform the second zero setting, the processor 512 may determine a second zero point position based on an angle of the first angle sensors 524 and 524-1 measured from the user's reference posture. The processor 512 may perform the second zero setting process for setting a zero point for the first angle sensors 524 and 524-1 based on the determined second zero point position.

In an embodiment, the processor 512 may set a zero point of an angle value output from the first angle sensors 524 and 524-1 when the first zero point position is determined through the first zero setting process and may set a zero point of an angle value output from the first angle sensors 524 and 524-1 based on the second zero point position when the second zero point position is determined through the second zero setting process described above. The first zero point position may correspond to an absolute zero point position determined by the second angle sensors 526 and 526-1, and the second zero point position may correspond to a relative zero point position determined according to the user's posture.

In an embodiment, if the first zero point position is determined after the second zero point position is determined, the processor 512 may perform zero setting for the first angle sensors 524 and 524-1 based on the first zero point position. If both the first zero point position and the second zero point position are determined, the first zero point position is preferential to the second zero point position, and zero setting may be performed on the first angle sensors 524 and 524-1 based on the first zero point position.

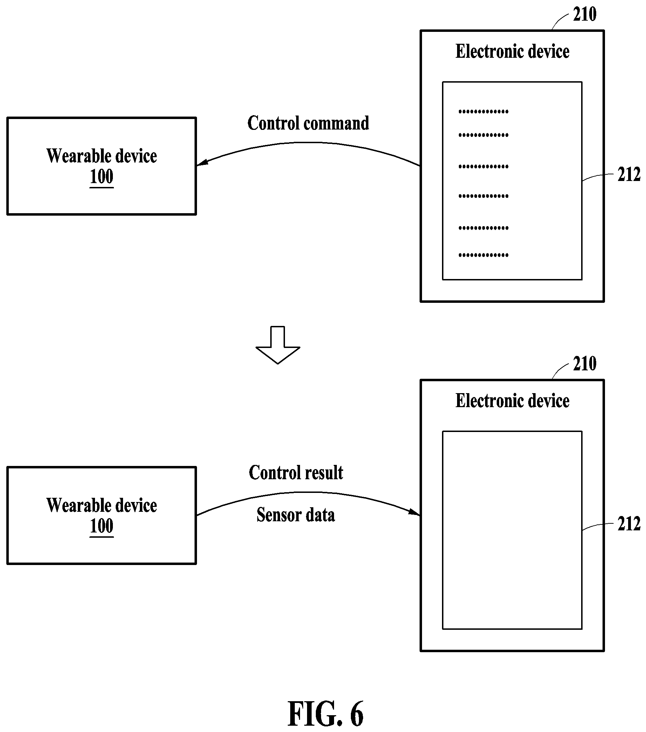

FIG. 6 is a diagram illustrating an interaction between a wearable device and an electronic device, according to an embodiment.

Referring to FIG. 6, the wearable device 100 may communicate with the electronic device 210. For example, the electronic device 210 may be a user terminal of a user using the wearable device 100 or a controller device dedicated to the wearable device 100. In an embodiment, the wearable device 100 and the electronic device 210 may be connected to each other through short-range wireless communication (e.g., Bluetooth^{™} or Wi-Fi communication).

In an embodiment, the electronic device 210 may check a state of the wearable device 100 or may execute an application to control or operate the wearable device 100. A screen of a user interface (UI) may be displayed to control the operation of the wearable device 100 or determine an operation mode of the wearable device 100 on a display 212 of the electronic device 210 through the execution of the application. The UI may be, for example, a GUI.

In an embodiment, the user may input an instruction (e.g., an instruction to execute an operation mode) for controlling the operation of the wearable device 100 or may change settings of the wearable device 100 through a GUI screen on the display 212 of the electronic device 210. The electronic device 210 may generate a control command (or a control signal) corresponding to an operation control command or setting change command input by the user and may transmit the generated control command to the wearable device 100. The wearable device 100 may operate according to the received control command and may transmit a control result according to the received control command and/or sensor data sensed by a sensor module of the wearable device 100 to the electronic device 210. The electronic device 210 may provide the user with result information (e.g., gait ability information, exercise ability information, or exercise result information) derived by analyzing the control result and/or the sensor data through the GUI screen.

In an embodiment, the electronic device 210 may transmit command data to perform zero setting (or calibration) on a sensor to the wearable device 100. The zero setting may include a zero setting process that initializes the sensor data output from the sensor (e.g., the first angle sensor 524 or the first angle sensor 524-1) of the wearable device 100. The zero setting process is a process corresponding to an initialization process for the sensor data output from the sensor of the wearable device 100 and a process for setting the sensor data output from the sensor to a reference value when the user takes a preparation posture to start an exercise or measure physical ability. Because a body state (e.g., a leg angle or a torso inclination) may vary depending on users in their preparation posture, a process for initializing the sensor data output from the sensor of the wearable device 100 in their preparation posture may be required to accurately measure the motion and/or posture of the users. When the zero setting process is completed, the wearable device 100 may notify the electronic device 210 of the completion of zero setting by transmitting notification data.

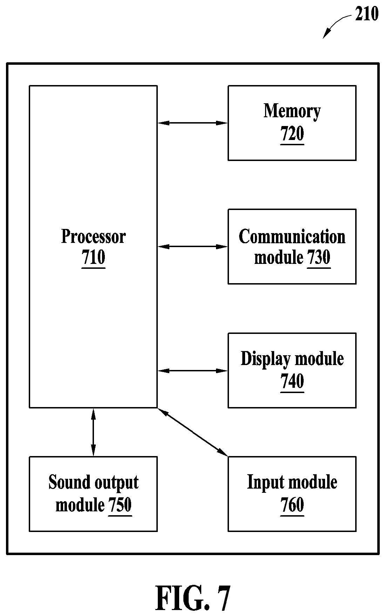

FIG. 7 is a diagram illustrating a configuration of an electronic device according to an embodiment.

Referring to FIG. 7, the electronic device 210 may include a processor 710, a memory 720, a communication module 730, a display module 740, a sound output module 750, and an input module 760. In an embodiment, at least one of the components (e.g., the sound output module 750) may be omitted from the electronic device 210, or one or more other components (e.g., a sensor module, a haptic module, and a battery) may be added to the electronic device 210.

The processor 710 may control at least one other component (e.g., a hardware or software component) of the electronic device 210 and may perform various types of data processing or operations. In an embodiment, as at least a part of data processing or operations, the processor 710 may store instructions or data received from another component (e.g., the communication module 730) in the memory 720, may process the instructions or the data stored in the memory 720, and may store result data in the memory 720.

In an embodiment, the processor 710 may include a main processor (e.g., a CPU or an AP) or an auxiliary processor (e.g., a GPU, an NPU, an ISP, a sensor hub processor, or a CP) that is operable independently of or in conjunction with the main processor.

The memory 720 may store various pieces of data used by at least one component (e.g., the processor 710 or the communication module 730) of the electronic device 210. The various pieces of data may include, for example, a program (e.g., an application) and input data or output data for a command related thereto. The memory 720 may include at least one instruction executable by the processor 710. The memory 720 may include a volatile memory or a non-volatile memory.

The communication module 730 may support establishing a direct (e.g., wired) communication channel or a wireless communication channel between the electronic device 210 and another electronic device (e.g., the wearable device 100, the other wearable devices 220, and the server 230) and performing communication via the established communication channel. The communication module 730 may include a communication circuit for performing a communication function. The communication module 730 may include one or more CPs that are operable independently of the processor 710 (e.g., an AP) and that support direct (e.g., wired) communication or wireless communication. In an embodiment, the communication module 730 may include a wireless communication module (e.g., a Bluetooth^{™} communication module, a cellular communication module, a Wi-Fi communication module, or a GNSS communication module) that performs wireless communication or a wired communication module (e.g., a LAN communication module or a power line communication (PLC) module). For example, the communication module 730 may transmit a control command to the wearable device 100 and may receive, from the wearable device 100, at least one of sensor data including body motion information of a user wearing the wearable device 100, state data of the wearable device 100, and control result data corresponding to the control command. The communication module 740 may transmit guide data and/or notification data to the other wearable device 220. The communication module 740 may transmit sensor data and user data received from the wearable device 100 to the server 230 and receive exercise result data and exercise program data from the server 230.

The display module 740 may visually provide information to the outside (e.g., the user) of the electronic device 210. The display module 740 may include, for example, a light-emitting diode (LCD) or organic light-emitting diode (OLED) display, a hologram device, or a projector device. The display module 740 may further include a control circuit to control the driving of the display. In an embodiment, the display module 740 may further include a touch sensor set to sense a touch or a pressure sensor set to sense the intensity of a force generated by the touch. The display module 740 may output a UI screen to control the wearable device 100 or provide various pieces of information (e.g., exercise evaluation information or setting information of the wearable device 100).

The sound output module 750 may output a sound signal to the outside of the electronic device 210. The sound output module 750 may include a guide sound signal (e.g., a driving start sound or an operation error notification sound) based on a state of the wearable device 100 and a speaker for playing musical content or a guide voice. When it is determined that the wearable device 100 is not properly worn on the body of the user, the sound output module 750 may output a guide voice to inform the user that they are wearing the wearable device 100 abnormally or to guide the user to wear the wearable device 100 normally. The sound output module 750 may output, for example, a guide voice corresponding to exercise evaluation information or exercise result information obtained by evaluating an exercise of the user.

The input module 760 may receive a command or data to be used by another component (e.g., the processor 710) of the electronic device 210 from the outside (e.g., the user) of the electronic device 210. The input module 760 may include an input component circuit and receive a user input. The input module 760 may include, for example, a touch recognition circuit for recognizing a touch on a key (e.g., a button) and/or a screen.

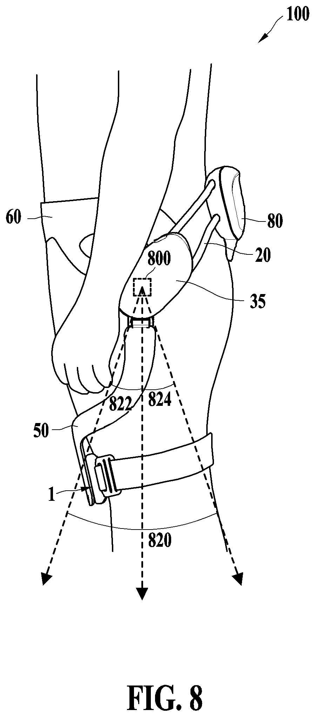

FIG. 8 is a diagram illustrating first zero setting using a second angle sensor according to an embodiment.

Referring to FIG. 8, the wearable device 100 may include a second angle sensor 800 (e.g., the second angle sensor 526-1) that measures an angle change of the second leg driving frame 50 based on a reference position in a cover of the second driving module 35. The wearable device 100 may include a second angle sensor (e.g., the second angle sensor 526) that measures an angle change of the first leg driving frame 55 on the opposite side based on the reference position in a cover of a first driving module 30. The description above may also apply to the performing of first zero point setting on the first angle sensor 524 by using the second angle sensor that measures the angle change of the first leg driving frame 55.

The second angle sensor 800 may be, for example, a hall sensor or an absolute encoder that is at the reference position. The reference position may correspond to an initial position that is a reference for controlling the operation of the first leg driving frame 50. The second angle sensor 800 may be toggled on when an angle formed by the first leg driving frame 50 with respect to the reference position is within a detection range 820 and may be toggled off when the angle is outside the detection range 820. The second angle sensor 800 is connected to a motor of the second driving module 35 and may measure an absolute position of the first leg driving frame 55 with respect to the reference position. The second angle sensor 800 may be used as a detection sensor for setting a zero point of the wearable device 100.

In the first zero setting, a zero point position may be set based on a sensor, such as the second angle sensor 800, mounted to a mechanically fixed position. When a user walks while wearing the wearable device 100, the relative angle difference between a forward angle change value 822 and a backward angle change value 824 based on a reference position of the second angle sensor 800 may be automatically calculated, and the zero point position for zero point setting may be determined based on the calculated relative angle difference.

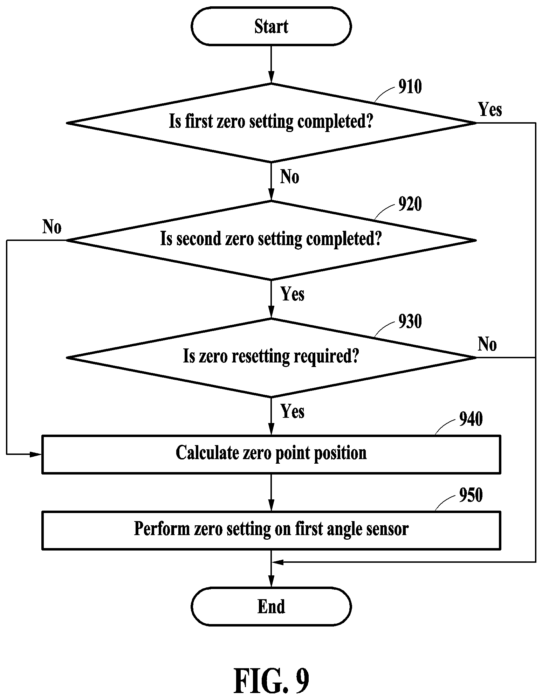

FIG. 9 is a flowchart illustrating an operation of a zero setting method of a wearable device, according to an embodiment. In an embodiment, at least one of the operations of FIG. 9 may be simultaneously or parallelly performed with one another, and the order of the operations may be changed. In addition, at least one of the operations may be omitted or another operation may be additionally performed.

Referring to FIG. 9, in operation 910, the wearable device 100 may identify whether first zero setting is completed for a first angle sensor (e.g., the first angle sensors 524 and 524-1) that measures an angle of a leg driving frame (e.g., the leg driving frames 50 and 55) included in the wearable device 100. The wearable device 100 may determine a first zero point position by using a second angle sensor (e.g., the second angle sensors 526 and 526-1) of the wearable device 100 and may determine whether the first zero setting is completed based on the first zero point position. If the first zero setting is identified to be completed ('Yes' in operation 910), a status quo may be maintained without a separate zero setting process.

If the first zero setting is identified to be not completed ('No' in operation 910), the wearable device 100 may identify whether second zero setting is completed in operation 920. For example, when the first zero point position may not be determined, and the first zero setting is not completed, the wearable device 100 may determine a second zero point position based on an angle value of the first angle sensor (e.g., the first angle sensors 524 and 524-1) of the wearable device 100 measured in a user's reference posture and may determine whether the second zero setting is completed based on the second zero point position.

If the second zero setting is identified to be completed ('Yes' in operation 920), the wearable device 100 may determine whether zero resetting is required for the first angle sensor in operation 930. The wearable device 100 may determine whether zero resetting is required based on a state change of the wearable device 100. The state change of the wearable device 100 may include, for example, a change in at least one of a connection state between the wearable device 100 and a peripheral device, a power state of the wearable device 100, an operation state of the wearable device 100, and the state of a driving module of the wearable device 100. In addition, the wearable device 100 may determine whether zero resetting is required based on whether an error occurs in the wearable device 100. Even if second zero setting has been already completed, the wearable device 100 may perform a zero setting process if the zero resetting is determined to be required based on the state change of the wearable device 100. If the zero resetting is determined to be not required ('No' in operation 930), a status quo may be maintained without a separate zero setting process.

If the second zero setting is identified to be not completed ('Yes' in operation 920) or the zero resetting is determined to be required ('Yes' in operation 930), the wearable device 100 may calculate a zero point position by performing a zero setting process in operation 940. In an embodiment, the wearable device 100 may perform a first zero setting process that calculates the first zero point position by using the second angle sensor for detecting the zero point position. In the first zero setting process, without requesting the reference posture from the user, a change of an angle formed by leg driving frames (e.g., the leg driving frames 50 and 55) of the wearable device 100 passing through the reference position of the second angle sensor and moving when the user walks may be sensed, and, based on the sensed change of the angle, the first zero point position may be calculated. The second angle sensor may measure an angle change of a leg driving frame in a first direction and an angle change thereof in a second direction. Based on the angle change in the first direction and the angle change in the second direction measured by the second angle sensor, the wearable device 100 may determine the first zero point position. For example, the wearable device 100 may determine the first zero point position based on first edge angle values and second edge angle values after extracting the first edge angle values in the first direction based on the angle change in the first direction and the second edge angle values in the second direction based on the angle change in the second direction. A first edge angle value may be an angle value measured when a leg driving frame (or the user's leg) turns from the first direction to the second direction among angle values measured by the second angle sensor when the leg driving frame of the wearable device 100 moves in the first direction. A second edge angle value may be an angle value measured when a leg driving frame (or the user's leg) changes from the second direction to the first direction among angle values measured by the second angle sensor when the leg driving frame of the wearable device 100 moves in the second direction.

If zero resetting is determined to be required based on the state change of the wearable device 100, and a zero point of the first angle sensor has been already set by the first zero setting process, a second zero setting process that performs zero setting based on the user's reference posture may not need to be performed. If the first zero point position is not determined by the first zero setting process within a defined time after the zero resetting is determined to be required, the second zero setting process may be performed.

In an embodiment, if the first zero point position may not be readily calculated through the first zero setting process (e.g., if normal angle measurement may not be readily performed through the second angle sensor based on the user's posture or the user's motion state), the second zero setting process may be performed based on a relative position of the user's posture. The second zero setting process may include requesting the reference posture for zero setting from the user and calculating the second zero point position based on the angle measured by the first angle sensor in the user's reference posture. In an embodiment, the wearable device 210 may guide the user to the reference posture for second zero setting through voice. Alternatively, the electronic device 210 may guide the user to the reference posture through voice or a display screen.

In operation 950, the wearable device 100 may perform zero setting of the first angle sensor based on the zero point position calculated in operation 940. The wearable device 100 may set a zero point for the first angle sensor based on the first zero point position or the second zero point position determined in operation 940. An output sensor value of the first angle sensor may be initialized to a sensor value of the zero point position through the zero setting.

As described above, the wearable device 100 may automatically determine a zero setting state, may determine whether zero setting is required, and may perform a zero setting process.

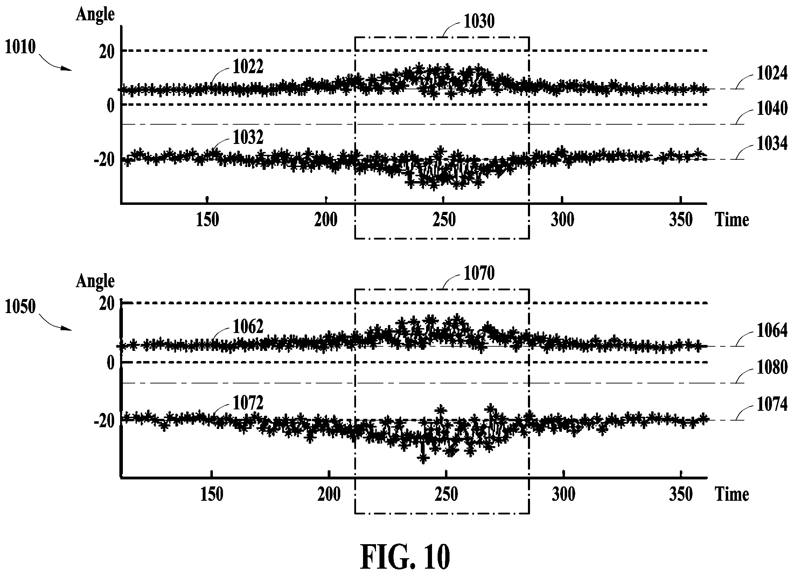

FIG. 10 is a diagram illustrating determining a first zero point position based on sensor data obtained from a second angle sensor, according to an embodiment.

Referring to FIG. 10, when a user walks while wearing the wearable device 100, graph 1010 illustrates edge angle values 1022 and 1032 measured by the second angle sensor 526-1 corresponding to a left leg, and graph 1050 illustrates edge angle values 1062 and 1072 measured by the second angle sensor 526 corresponding to a right leg. Graph 1010 illustrates first edge angle values 1032 an angle change in a first direction (e.g., a forward direction) and second edge angle values 1022 of an angle change in a second direction (e.g., a backward direction), and graph 1050 illustrates first edge angle values 1072 of the angle change in the first direction (e.g., the forward direction) and second edge angle values 1062 of the angle change in the second direction (e.g., the backward direction).

In an embodiment, with a predetermined sensing range (e.g., an angle range of - 12 to +12 degrees based on a reference position) for the second angle sensors 526 and 526-1 to sense a spacing angle from the reference position, the wearable device 100 may detect a falling edge and a rising edge in the sensor data of the second angle sensors 526 and 526-1 measured within the sensing range and may calculate the first zero point position based on the angles of the detected falling edge and rising edge. The second angle sensors 526 and 526-1 may measure the angles of a falling edge and a rising edge generated when the leg driving frames 50 and 55 of the wearable device 100 pass through the sensing range of the second angle sensors 526 and 526-1 and a non-sensing range (a range outside the sensing range) thereof and move.

In an embodiment, the angle deviation of the second angle sensors 526 and 526-1 may increase in a section where the user's motion speed increases. Thus, only sensor data detected at a specified motion speed or less may be used to calculate the first zero point position. For example, edge angle values in a section 1030 among the edge angle values 1022 and 1032 may not be used to calculate the first zero point position of the first angle sensor 524-1 corresponding to the left leg. Likewise, edge angle values in a section 1070 among the edge angle values 1062 and 1072 may not be used to calculate the first zero point position of the first angle sensor 524 corresponding to the right leg. The edge angle values in the section 1030 and the edge angle values in the section 1070 may be treated as noise and removed. For example, the wearable device 100 may store edge angle values of a falling edge at the speed of 120 degrees per second (deg/s) or less and then may extract a moving minimum/maximum (moving min/max) value of the stored edge angle values. The first zero point position may be determined based on the moving min/max value considered an accurate edge angle value. For example, when a moving min/max value 1024 of the edge angle values 1022 and a moving min/max value 1034 of the edge angle values 1032 are determined, a median position angle value 1040 between the moving min/max value 1024 and the moving min/max value 1034 may be determined to be the first zero point position for the zero setting of the first angle sensor 524-1. Likewise, when a moving min/max value 1064 of the edge angle values 1062 and a moving min/max value 1074 of the edge angle values 1072 are determined, a median position angle value 1080 between the moving min/max value 1064 and the moving min/max value 1074 may be determined to be the first zero point position for the zero setting of the first angle sensor 524.

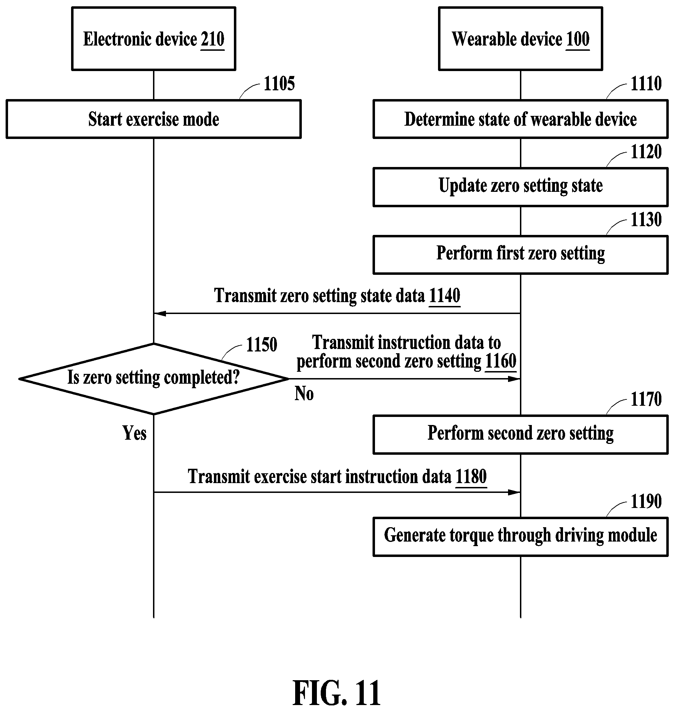

FIG. 11 is a flowchart illustrating an operation of a zero setting method performed between an electronic device and a wearable device, according to an embodiment. In an embodiment, at least one of operations of FIG. 11 may be simultaneously or parallelly performed with one another, and the order of the operations may be changed. In addition, at least one of the operations may be omitted or another operation may be additionally performed.

Referring to FIG. 11, in operation 1105, the electronic device 210 may start an exercise mode. When a user inputs an instruction to start exercise through an application of the electronic device 210 or is estimated to start the exercise mode (e.g., a walking motion), the exercise mode may be started. When the exercise mode is started, the electronic device 210 may transmit notification data to notify the start of the exercise mode to the wearable device 100.

In operation 1110, the wearable device 100 may determine the state of the wearable device 100. The wearable device 100 may periodically determine the current state of the wearable device 100. For example, the wearable device 100 may detect a change in at least one of a connection state between the wearable device 100 and a peripheral device, a power state of the wearable device 100, an operation state of the wearable device 100, and the state of a driving module of the wearable device 100. Based on the current state of the wearable device 100, the wearable device 100 may determine whether zero resetting is required for a first angle sensor configured to measure an angle of a leg driving frame included in the wearable device 100. In an embodiment, when the state change of the wearable device 100 is detected, the wearable device 100 may control to initialize a zero setting state and reperform zero setting.

In operation 1120, the wearable device 100 may update the zero setting state of the wearable device 100. The wearable device 100 may include, as the updated zero setting state, information on whether a first zero point position is determined through a first zero setting process, whether a second zero point position is determined through a second zero setting process, and whether zero resetting is currently required. When the exercise mode is started, zero setting state data may be periodically transmitted to the electronic device 210.

In operation 1130, the wearable device 100 may perform the first zero setting process that determines the first zero point position by using a second angle sensor when first zero setting is not completed. In an embodiment, in response to a determination that zero resetting is required, by using the second angle sensor configured to measure an angle change in a first direction and an angle change in a second direction of the leg driving frame, the wearable device 100 may perform the first zero setting process for a first angle sensor. The first zero setting process may be automatically performed by the wearable device 100 without notifying the user of performing zero setting. The wearable device 100 may determine the first zero point position based on the angle change in the first direction and the angle change in the second direction measured by the second angle sensor and may set a zero point for the first angle sensor based on the determined first zero point position. The wearable device 100 may determine the first zero point position based on an angle value (corresponding to an edge angle value) when the user's leg turns from the first direction to the second direction and an angle value (corresponding to an edge angle value) when the user's leg turns from the second direction to the first direction, which are measured when the user walks while wearing the wearable device 100. When the first zero point position is determined, the wearable device 100 may set the first zero point position as the zero point of the first angle sensor. If the second zero point position is set through the second zero setting before the first zero point position is determined, the wearable device 100 may initialize the second zero point position and may set the zero point of the first angle sensor to the determined first zero point position.

If the first zero setting process may not be performed (e.g., if the normal determination of the first zero point position may not be readily performed from the sensor data of the second angle sensor based on the user's posture or motion), operation 1130 may be omitted.

In operation 1140, the wearable device 100 may transmit zero setting state data indicating a zero setting state of the wearable device 100 to the electronic device 210. When an exercise mode is started, zero setting state data may be periodically transmitted to the electronic device 210.

In operation 1150, based on the zero setting state data, the electronic device 210 may determine whether zero setting for the first angle sensor of the wearable device 100 is completed. If the zero setting of the wearable device 100 is determined to be completed ('Yes' in operation 1150), the electronic device 210 may transmit exercise start instruction data to the wearable device 100 in operation 1180. In operation 1190, in response to receiving the exercise start instruction data, the wearable device 100 may generate torque through a driving module. The wearable device 100 may estimate the user's leg motion and/or exercise state based on the sensor data of the first angle sensor after the zero setting is completed and may adjust the size of torque based on the estimated result.

If the zero setting of the wearable device 100 is determined to be not completed ('No' in operation 1150), the electronic device 210 may induce the user to take a reference posture for zero setting in operation 1160 and may transmit instruction data to perform second zero setting the wearable device 100.

In operation 1170, in response to receiving command data to perform second zero setting from the electronic device 210, the wearable device 100 may perform the second zero setting process for the first angle sensor by using the first angle sensor. The wearable device 100 may determine the second zero point position based on the angle of the first angle sensor measured in the user's reference posture and may set a zero point for the first angle sensor based on the determined second zero point position. For example, the wearable device 100 may determine an angle value of the first angle sensor measured in the user's reference posture to be the second zero point position. The reference posture may vary depending on the types of exercise selected to be performed by the user. After the second zero setting process is completed, the wearable device 100 may generate torque through the driving module in operation 1190.

As described above, the process of performing zero setting on the wearable device 100 may use the first zero setting process using an absolute angle value output from the second angle sensor and the second zero setting process using a relative angle value measured in the user's reference posture. The wearable device 100 may not perform a zero setting process if zero resetting is not required by determining whether the zero resetting is required based on the state of the wearable device 100, and, even if the zero resetting is required, may reduce the number of performing the second zero setting process by preferentially performing the first zero setting process that is automatically performed by the wearable device 100 prior to the second zero setting process. The convenience of using the wearable device 100 may be improved by reducing the number of second zero setting processes requiring the user's active participation.

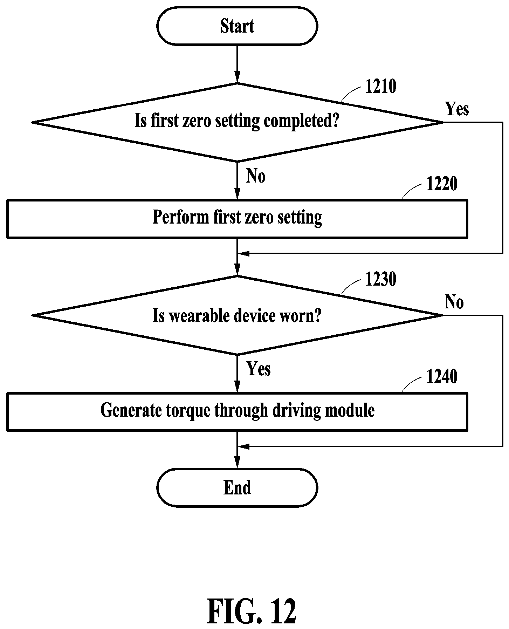

FIG. 12 is a flowchart illustrating a first zero setting process that is automatically performed by a wearable device, according to an embodiment. According to an embodiment, the first zero setting process may be performed by the wearable device 100 without requesting the reference posture for zero setting from the user or using the electronic device 210. In an embodiment, at least one of operations of FIG. 12 may be simultaneously or parallelly performed with one another, and the order of the operations may be changed. In addition, at least one of the operations may be omitted or another operation may be additionally performed.

Referring to FIG. 12, in operation 1210, the wearable device 100 may identify whether first zero setting is completed for a first angle sensor (e.g., the first angle sensors 524 and 524-1) that measures an angle of a leg driving frame (e.g., the leg driving frames 50 and 55) included in the wearable device 100. The wearable device 100 may determine a first zero point position by using a second angle sensor (e.g., the second angle sensors 526 and 526-1) of the wearable device 100 and may determine whether the first zero setting is completed based on the first zero point position.

If the first zero setting is identified to be not completed ('No' in operation 1210), the wearable device 100 may perform the first zero setting by using the second angle sensor for detecting a zero point position in operation 1220. The wearable device 100 may automatically perform the first zero setting process without a second zero setting process. In the first zero setting process, without requesting the reference posture from the user, a change of an angle formed by leg driving frames (e.g., the leg driving frames 50 and 55) of the wearable device 100 passing through the reference position of the second angle sensor and moving when the user walks may be sensed, and, based on the sensed change of the angle, the first zero point position may be calculated. The wearable device 100 may calculate the first zero point position corresponding to an absolute zero point position based on a change in a sensor value of the second angle sensor based on the motion of the user's leg. The wearable device 100 may perform zero setting of the first angle sensor based on the calculated first zero point position. An output sensor value of the first angle sensor may be initialized to a sensor value of the first zero point position through the zero setting.

If the first zero setting is identified to be completed in operation 1210 ('Yes' in operation 1210) or the performing of the first zero setting is completed in operation 1220, the wearable device 100 may determine whether the user properly wears the wearable device 100 in operation 1230. In an embodiment, the wearable device 100 may determine a movement score indicating how much the wearable device 100 moves during a time interval based on sensor data obtained by a sensor (e.g., an inertia sensor or a first angle sensor) included in the wearable device 100 and may determine that the user properly wears the wearable device 100 if the movement score is greater than or equal to a threshold value. If the determined movement score is less than the threshold value, it may be determined that the user does not properly wear the wearable device 100.

If it is determined that the user does not properly wear the wearable device 100 ('No' in operation 1230), the wearable device 100 may not output torque. In addition, the wearable device 100 may output a guide voice to induce the user to properly wear the wearable device 100. The wearable device 100 may not output torque even if the user requests to start its operation until the proper wearing of the wearable device 100 is confirmed.

If it is determined that the user properly wears the wearable device 100 ('Yes' in operation 1230), the wearable device 100 may generate torque through the driving modules 530 and 530-1 of the wearable device 100 in operation 1240. The wearable device 100 may estimate the user's current leg motion and/or the user's exercise state (e.g., a walking phase, an exercise motion, or an exercise step) based on the sensor data of the first angle sensor obtained after the first zero setting. The wearable device 100 may calculate the size of torque suitable for the current exercise state based on the estimated exercise state of the user and may output torque of the calculated size through the driving module.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things, unless the relevant context clearly indicates otherwise. As used herein, "A or B", "at least one of A and B", "at least one of A or B", "A, B or C", "at least one of A, B and C", and "A, B, or C," each of which may include any one of the items listed together in the corresponding one of the phrases, or all possible combinations thereof. Terms such as "first", "second", or "first" or "second" may simply be used to distinguish the component from other components in question, and do not limit the components in other aspects (e.g., importance or order). It is to be understood that if an element (e.g., a first element) is referred to, with or without the term "operatively" or "communicatively," as "coupled with," "coupled to," "connected with," or "connected to" another element (e.g., a second element), the element may be coupled with the other element directly (e.g., by wire), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

The software may include a computer program, a piece of code, an instruction, or some combination thereof, to independently or collectively instruct or configure the processing device to operate as desired. Software and data may be embodied permanently or temporarily in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording mediums. Embodiments as set forth herein may be implemented as software including one or more instructions that are stored in a storage medium (e.g., the memory 514) that is readable by a machine. For example, a processor of the machine may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to embodiments of the disclosure may be included and provided in a computer program product. The computer program product may be traded as a product between a seller and a buyer. The computer program product may be distributed in the form of a machine-readable storage medium (e.g., a compact disc read-only memory (CD-ROM)), or be distributed (e.g., downloaded or uploaded) online via an application store (e.g., PlayStore^{™}), or between two user devices (e.g., smartphones) directly. If distributed online, at least part of the computer program product may be temporarily generated or at least temporarily stored in the machine-readable storage medium, such as a memory of the manufacturer's server, a server of the application store, or a relay server.

According to an embodiment, each component (e.g., a module or a program) of the above-described components may include a single entity or multiple entities, and some of the multiple entities may be separately disposed in different components. According to an embodiment, one or more of the above-described components may be omitted, or one or more other components may be added. Alternatively or additionally, a plurality of components (e.g., modules or programs) may be integrated into a single component. In such a case, according to an embodiment, the integrated component may still perform one or more functions of each of the components in the same or similar manner as they are performed by a corresponding one among the components before the integration. According to embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable device 100 worn on a user's body, the wearable device 100 comprising:
a driving module 530; 530-1 configured to generate torque applied to the user's body;
a leg driving frame 50; 55 configured to relay the generated torque to the user's leg;
a thigh fastener 1; 2 that is connected to the leg driving frame 50; 55 and configured to fix the leg driving frame 50; 55 to the user's leg;
a sensor module 520 configured to obtain sensor data comprising motion information of the wearable device 100; and
a processor 512 configured to perform zero setting based on the sensor data on a sensor comprised in the wearable device 100,
wherein the sensor module 520 comprises a first angle sensor 524; 524-1 configured to measure an angle of the leg driving frame 50; 55 and a second angle sensor 526; 526-1 configured to measure an angle change in a first direction and an angle change in a second direction of the leg driving frame 50; 55 based on a reference position in a sensing range, and
the processor 512 is further configured to determine a first zero point position based on the angle change in the first direction and the angle change in the second direction measured by the second angle sensor 526; 526-1 and perform a first zero setting process for setting a zero point for the first angle sensor 524; 524-1 based on the determined first zero point position.

2. The wearable device 100 of claim 1, wherein
the processor 512 is further configured to
determine whether zero resetting is required based on a state change of the wearable device 100 and control to perform the first zero setting process in response to a determination that the zero resetting is required.

3. The wearable device 100 of claim 2, wherein
the state change of the wearable device 100 comprises:
a change in at least one among a connection state between the wearable device 100 and a peripheral device, a power state of the wearable device 100, an operation state of the wearable device 100, and a state of the
driving module 530; 530-1.

4. The wearable device 100 of any one of claims 1 to 3, wherein
the processor 512 is further configured to
determine the first zero point position based on an angle when the user's leg turns from the first direction to the second direction and an angle when the user's leg turns from the second direction to the first direction, which are measured when the user walks while wearing the wearable device 100.

5. The wearable device 100 of any one of claims 1 to 4, wherein
the first zero setting process is
automatically performed by the wearable device 100 without notifying the user of performing zero setting.

6. The wearable device 100 of any one of claims 1 to 5, further comprising:
a communication module 516 configured to wirelessly communicate with an electronic device 210,
wherein the communication module 516 is further configured to periodically transmit zero setting state data indicating a zero setting state of the wearable device 100 to the electronic device 210.

7. The wearable device 100 of claim 6, wherein
the electronic device 210 is configured to
transmit command data to perform second zero setting to the wearable device 100 if the first zero point position of the wearable device 100 is identified as undetermined, based on the zero setting state data.

8. The wearable device 100 of claim 7, wherein
the processor 512 is further configured to,
in response to receiving the command data to perform the second zero setting from the electronic device 210, a second zero point position is determined based on an angle of the first angle sensor 524; 524-1 measured from the user's reference posture, perform a second zero setting process for setting a zero point for the first angle sensor 524; 524-1 based on the determined second zero point position.

9. The wearable device 100 of claim 8, wherein
the processor 512 is further configured to,
if the first zero point position is determined after the second zero point position is determined, perform zero setting for the first angle sensor 524; 524-1 based on the first zero point position.

10. The wearable device 100 of claim 8, wherein
the processor 512 is further configured to,
if the first zero point position is determined by the first zero setting process, set a zero point of an angle value that is output from the first angle sensor 524; 524-1 based on the first zero position, and,
if the second zero point position is determined by the second zero setting process, set a zero point of an angle value that is output from the first angle sensor 524; 524-1 based on the second zero position.

11. A zero setting method performed by a wearable device 100, the zero setting method comprising:
determining whether zero resetting is required for a first angle sensor 524; 524-1 configured to measure an angle of a leg driving frame 50; 55 comprised in the wearable device 100; and,
in response to a determination that the zero resetting is required, by using a second angle sensor 526; 526-1 configured to measure an angle change in a first direction and an angle change in a second direction of the leg driving frame 50; 55, performing a first zero setting process for the first angle sensor 524; 524-1,
wherein the performing of the first zero setting process comprises:
determining a first zero point position based on the angle change in the first direction and the angle change in the second direction measured by the second angle sensor 526; 526-1; and
setting a zero point for the first angle sensor 524; 524-1 based on the determined first zero point position.

12. The zero setting method of claim 11, wherein
the determining of whether the zero resetting is required comprises:
determining whether the zero resetting is required based on a state change of the wearable device 100, and
the state change of the wearable device 100 comprises:
a change in at least one among a connection state between the wearable device 100 and a peripheral device, a power state of the wearable device 100, an operation state of the wearable device 100, and a state of the driving module 530; 530-1.

13. The zero setting method of claim 11 or 12, wherein
the determining of the first zero point position comprises:
determining the first zero point position based on an angle when a user's leg turns from the first direction to the second direction and an angle when the user's leg turns from the second direction to the first direction, which are measured when the user walks while wearing the wearable device 100.

14. The zero setting method of any one of claims 11 to 13, further comprising: transmitting zero setting state data indicating a zero setting state of the wearable device 100 to an electronic device 210; and,
in response to receiving command data to perform second zero setting from the electronic device 210, performing a second zero setting process for the first angle sensor 524; 524-1 by using the first angle sensor 524; 524-1,
wherein the performing of the second zero setting process comprises:
determining the second zero point position based on an angle of the first angle sensor 524; 524-1 measured from the user's reference posture; and setting a zero point for the first angle sensor 524; 524-1 based on the determined second zero point position.

15. Anon-transitory computer-readable storage medium storing instructions that, when executed by a processor, cause the processor to perform the zero setting method of any one of claims 11 to 14.
